# EUROPÄISCHE PATENTSCHRIFT

(11) **EP 2 801 382 B1**
(45) Veröffentlichungstag und Bekanntmachung des Hinweises auf die Patenterteilung: **06.06.2018**
(21) Anmeldenummer: 14165994.6
(22) Anmeldetag: 25.04.2014
(51) Int. Cl.: A61M 5/315

(54) **Dosiersystem**
Metering system
Système de dosage

(30) Priorität: 07.05.2013 DE 102013208390
(43) Veröffentlichungstag der Anmeldung: 12.11.2014
(73) Patentinhaber: RAUMEDIC AG, 95213 Münchberg (DE)
(72) Erfinder: Skaper, Frank, 95191 Leupoldsgrün (DE); Eichelkraut, Gero, 01279 Dresden (DE); Braun, Thomas, 95032 Hof (DE); Spindler, Bernd, 95233 Helmbrechts (DE); Witzel, Dirk, 36124 Eichenzell (DE); Krimmel, Mirko, 36088 Hünfeld (DE); Vogt, Andreas, 36419 Geisa (DE)
(74) Vertreter: Rau, Schneck & Hübner Patentanwälte Rechtsanwälte PartGmbB

(56) Entgegenhaltungen:
- EP-A1- 1 459 776
- EP-A1- 2 570 145
- US-A1- 2012 316 509

## Beschreibung

Die Erfindung betrifft ein Dosiersystem zur Einstellung eines Dosiervolumens eines fließfähigen Mediums, wie eines flüssigen Medikaments, gemäß dem Oberbegriff des Anspruchs 1.

Aus dem Stand der Technik sind Dosiersysteme bekannt, deren Dosiervolumen einstellbar sind. Diese Dosiersysteme umfassen im Allgemeinen jeweils eine Dosierhülse und einen in der Dosierhülse translatorisch verschiebbar geführten Dosier-Kolbenkörper. Zur Einstellung des jeweiligen Dosiervolumens werden an den Dosier-Kolbenkörpern umfangsseitig äußere Anschlagelemente in der gewünschten Dosierstellung angebracht, die ein entsprechendes Fenster in der Dosierhülse nach außen durchsetzen. Nachteilig an diesen bekannten Dosiersystemen ist, dass deren Handhabung oftmals nicht bedienerfreundlich ist. Ferner ist die Handhabung nicht immer intuitiv.

Die DE 355 954 B beschreibt eine Spritze für ärztliche Zwecke. Die DE 1 035 858 A und die US 5 183 466 beschreiben verschiedene Ausführungen einer Injektionsspritze.

Aus der EP 2 570 145 A1 ist eine gattungsgemäße Spritze bekannt. Die Spritze umfasst einen in einer Hülse verschiebbaren Kolbenkörper mit einem distalen Element und einem proximalen Element. Das distale Element ist mit Aussparungen versehen. Das proximale Element hat einen flexiblen Arm mit einem Vorsprung. Wenn ein Benutzer den Arm radial nach innen auslenkt, gelangt der Vorsprung außer Eingriff mit der entsprechenden Aussparung und das distale Element und das proximale Element sind dann in einer axialen Richtung der Spritze relativ zueinander verlagerbar. Die gewählte Aussparung wirkt sich auf die abgebbare Menge des Mittels aus.

Die US 2012/0316509 A1 offenbart Abgabesysteme zum Abgeben eines Fluidmittels. Ein Abgabesystem umfasst ein Gehäuse, in dem eine Kolbeneinheit verschiebbar geführt ist. In dem Gehäuse ist ein seitliches Fenster angeordnet, in das ein Einsatzelement einsetzbar ist. Das Einsatzelement ist in eingesetztem Zustand in dem seitlichen Fenster örtlich festgelegt. Es ist dann im Stande, die Kolbeneinheit daran zu hindern, vollständig aus dem Gehäuse gezogen zu werden.

Die EP 1 459 776 A1 offenbart Spritzen, mit welchen genaue Flüssigkeitsmengen abgebbar sind. Eine Spritze umfasst ein Behältnis und ein in dem Behältnis verschiebbar geführtes Kolbenelement. Ein Stoppelement ist entlang der Längsrichtung des Kolbenelements verschiebbar an diesem angeordnet. Die Position des Stoppelements an dem Kolbenelement ist über Fixiermittel einstellbar.

Der Erfindung liegt daher die Aufgabe zugrunde, ein Dosiersystem zur Einstellung eines Dosiervolumens eines fließfähigen Mediums bereitzustellen, das äußerst bedienerfreundlich und intuitiv ist. Ferner soll das Dosiersystem manipulationssicher sein und einfach herstellbar sein.

Diese Aufgabe wird erfindungsgemäß durch die in dem Anspruch 1 angegebenen Merkmale gelöst. Der Kern der Erfindung liegt darin, dass das Aufzieh-Begrenzungselement in seiner inneren Stellung vollständig bzw. in seiner Gesamtheit in der Dosierhülse angeordnet ist. Da sich das Aufzieh-Begrenzungselement in seiner inneren Stellung vollständig innerhalb der Dosierhülse befindet, ist so eine ungewollte Manipulation an dem Aufzieh-Begrenzungselement bzw. an dem Aufziehvorgang erschwert bzw. unmöglich gemacht. Das Aufzieh-Begrenzungselement ist in seiner inneren Stellung durch die Dosierhülse geschützt. Der Dosier-Kolbenkörper ist relativ zu der Dosierhülse verschiebbar, wenn sich das Aufzieh-Begrenzungselement in seiner inneren Stellung befindet. Der Dosier-Kolbenkörper ist auch relativ zu der Dosierhülse verschiebbar, wenn sich das Aufzieh-Begrenzungselement in seiner äußeren Stellung befindet.

Vorzugsweise kommt das Dosiersystem in der Kindermedizin oder bei der Behandlung alter Menschen zum Einsatz.

Wie bereits erwähnt, ist das Aufzieh-Begrenzungselement in seiner inneren Stellung vollständig innerhalb der Dosierhülse angeordnet. Die Dosierhülse kann dabei bzw. dennoch mindestens eine umfangsseitige Öffnung, Durchbrechung, ein Fenster oder dergleichen aufweisen, was auf diese Betrachtung der vollständig inneren Anordnung keinen Einfluss hat. Das Aufzieh-Begrenzungselement ist in der inneren Stellung in dem Aufnahmeraum der Dosierhülse angeordnet.

Die Dosierhülse begrenzt nach außen einen Aufnahmeraum für das abzugebende Medium. Die mindestens eine Kommunizierungsöffnung erlaubt ein Eintreten des Mediums in den Aufnahmeraum. Ferner ist das in dem Aufnahmeraum befindliche Medium über die mindestens eine Kommunizierungsöffnung wieder aus dem Aufnahmeraum abgebbar.

In der aufgezogenen Stellung des Dosier-Kolbenkörpers ist der Aufnahmeraum in seinem Volumen maximal, während er in der vorgerückten Stellung des Dosier-Kolbenkörpers dagegen minimal ist.

Es ist von Vorteil, wenn das Aufzieh-Begrenzungselement senkrecht zu der Längsmittelachse zwischen seinen Stellungen verlagerbar ist. Eine schräge oder rotatorische Verlagerung gegenüber der Längsmittelachse ist alternativ möglich.

In der aufgezogenen Stellung des Dosier-Kolbenkörpers schlägt das sich in der inneren Stellung befindende Aufzieh-Begrenzungselement an dem Anschlag an und begrenzt so zusammen mit dem Anschlag das Dosiervolumen. Das Aufzieh-Begrenzungselement bildet so einen Gegenanschlag für den Anschlag, der somit quasi einen Dosieranschlag bildet. Durch den taktilen bzw. mechanischen Anschlag ist im Gegensatz zu rein visuellen Einstellungen eine Überdosierung unmöglich. Bei herkömmlichen Dosierspritzen liegen fehlerhafte Dosierungen durchschnittlich bei 35 % bezogen auf die Dosiermenge, während bei üblichen Dosierlöffeln fehlerhafte Dosierungen durchschnittlich bei 85 % bezogen auf die Dosiermenge liegen. Der taktile Anschlag führt also zu einer erhöhten Sicherheit, was insbesondere bei hochaktiven Medikamenten und geringen Dosiervolumina vorteilhaft ist. Auch visuell beeinträchtigte Menschen können so eine genaue Dosierung einstellen bzw. vornehmen.

Das Aufzieh-Begrenzungselement ist günstigerweise in mindestens zwei verschiedenen, in Richtung der Längsmittelachse voneinander beabstandeten Positionen an dem Dosier-Kolbenkörper axial festlegbar. Bevorzugter ist das Aufzieh-Begrenzungselement in mindestens fünf, bevorzugter in mindestens zehn, bevorzugter in mindestens fünfzehn, verschiedenen, in Richtung der Längsmittelachse voneinander beabstandeten Positionen an dem Dosier-Kolbenkörper axial festlegbar. Durch diese Ausgestaltung ist eine äußerst feine Dosierinkrementierung mit vielen Dosierinkrementen geschaffen. Nachdem das Aufzieh-Begrenzungselement an dem inneren Dosier-Kolbenkörper axial festlegbar ist, liegt eine Innen-Dosierinkrementierung vor, was zu einer kompakten Bauform des Dosiersystems führt.

Ferner steht bei dem Dosiersystem im Wesentlichen der komplette Aufziehweg bzw. Hubweg für das Aufdosieren des zu dosierenden Mediums zur Verfügung. Insbesondere wird der Hubweg nicht durch einen Anschlag verbraucht bzw. reduziert.

Das Aufzieh-Begrenzungselement ist vorzugsweise klammerartig ausgeführt. Es ist zweckmäßig, wenn das Aufzieh-Begrenzungselement symmetrisch, bevorzugter U-förmig, ausgeführt ist.

Es ist von Vorteil, wenn an der Dosierhülse eine Dosierskalierung angebracht ist.

Günstigerweise ist das Dosiersystem spritzenartig ausgeführt. Das Design orientiert sich vorzugsweise an bekannten Dosierspritzen, die Benutzern geläufig sind. Das Dosiersystem ist vorzugsweise äußerst kompakt ausgeführt.

Es ist von Vorteil, wenn das Dosiersystem keine losen Teile hat. Die Teile des Dosiersystems sind dann verliersicher.

Es ist von Vorteil, wenn das Aufzieh-Begrenzungselement einen Betätigungskopf zur manuellen Verlagerung des Aufzieh-Begrenzungselements zwischen der äußeren Stellung und der inneren Stellung aufweist. Der Betätigungskopf erlaubt eine einfache und sichere Verlagerung des Aufzieh-Begrenzungselements zwischen der äußeren Stellung und der inneren Stellung, bevorzugter von der äußeren Stellung in die innere Stellung. Besonders bevorzugt ist die Verlagerung reversibel oder alternativ irreversibel von der äußeren Stellung in die innere Stellung.

Das Dosiersystem ist äußerst kostengünstig und einfach herstellbar.

Weitere vorteilhafte Ausgestaltungen der Erfindung sind in den Unteransprüchen angegeben.

Gemäß Anspruch 2 ist eine maximale Quererstreckung des Aufzieh-Begrenzungselements kleiner als der Innendurchmesser der Dosierhülse. Alle Erstreckungen des Aufzieh-Begrenzungselements senkrecht zu der Längsmittelachse sind somit kleiner als der Innendurchmesser der Dosierhülse, genauer betrachtet als der Innendruchmesser des Hauptzylinders der Dosierhülse.

Die Ausgestaltung nach Anspruch 3 erlaubt eine sichere und einfache örtliche axiale Festlegung des Aufzieh-Begrenzungselements an dem Dosier-Kolbenkörper. Eine radiale Festlegung ist zusätzlich durch eine formschlüssige Verbindung zwischen dem Aufzieh-Begrenzungselement und dem Dosier-Kolbenkörper möglich. Anstelle einer Rastverbindung kann beispielsweise auch eine Steckverbindung vorliegen. Die formschlüssige Verbindung in der inneren Stellung des Aufzieh-Begrenzungselements kann einmalig bzw. unlösbar verrastend oder wieder aufhebbar sein, was eine Demontage des Dosier-Kolbenkörpers von der Dosierhülse bzw. eine Entfernung des Dosier-Kolbenkörpers von der Dosierhülse ermöglicht. Eine derartige Demontage bzw. Entfernung ist beispielsweise zu Reinigungszwecken vorteilhaft.

Gemäß Anspruch 4 sind die ersten Formschlussmittel in einer Formschlussmittelleiste hintereinander angeordnet. Die ersten Formschlussmittel sind vorzugsweise außenseitig an dem Dosier-Kolbenkörper vorgesehen. Es ist von Vorteil, wenn das mindestens eine zweite Formschlussmittel innenseitig an dem Aufzieh-Begrenzungselement angeordnet ist. Die ersten und zweiten Formschlussmittel sind komplementär zueinander ausgebildet. Vorzugsweise ist eine Rast- oder Steckverbindung zwischen den Formschlussmitteln vorgesehen.

Die Ausgestaltung nach Anspruch 5 verhindert eine ungewollte Manipulation an dem Dosiersystem und somit eine ungewünschte Verstellung des Dosiervolumens. Es ist von Vorteil, wenn das Aufzieh-Begrenzungselement in der inneren Stellung sowohl axial als auch senkrecht zu der Längsmittelachse örtlich festgelegt ist. Wie bereits erwähnt, ist jedoch auch alternativ eine lösbare Verbindung zwischen dem sich in der inneren Stellung befindenden Aufzieh-Begrenzungselement und dem Dosier-Kolbenkörper möglich, was beispielsweise für Reinigungszwecke vorteilhaft ist.

Vorzugsweise ist an dem Dosier-Kolbenkörper mindestens ein erstes Rastelement angeordnet, das sich in Richtung der Längsmittelachse erstreckt. Bevorzugter sind an dem Dosier-Kolbenkörper mehrere erste Rastelemente angeordnet, die in Richtung der Längsmittelachse beabstandet zueinander angeordnet sind. Günstigerweise ist an dem Aufzieh-Begrenzungselement mindestens ein erstes Rastgegenelement angeordnet, das in der inneren Stellung des Aufzieh-Begrenzungselements mit mindestens einem der ersten Rastelemente in Rastverbindung steht und eine radiale Bewegung des Aufzieh-Begrenzungselements zu der Längsmittelachse nach außen erschwert oder unterbindet.

Vorzugsweise ist an dem Dosier-Kolbenkörper mindestens ein zweites Rastelement angeordnet, das sich in Richtung der Längsmittelachse erstreckt. Bevorzugter sind an dem Dosier-Kolbenkörper mehrere zweite Rastelemente angeordnet, die in Richtung der Längsmittelachse beabstandet zueinander angeordnet sind. Günstigerweise ist an dem Aufzieh-Begrenzungselement mindestens ein zweites Rastgegenelement angeordnet, das in der inneren Stellung des Aufzieh-Begrenzungselements mit mindestens einem der zweiten Rastelemente in Rastverbindung steht und eine radiale Bewegung des Aufzieh-Begrenzungselements zu der Längsmittelachse nach außen erschwert oder unterbindet.

Wenn sich das Aufzieh-Begrenzungselement in seiner inneren Stellung befindet, steht das Aufzieh-Begrenzungselement mit dem Dosier-Kolbenkörper in Eingriff, bevorzugter in Rasteingriff, der eine radiale Verlagerung des Aufzieh-Begrenzungselements nach außen verhindert. Diese innere Stellung des Aufzieh-Begrenzungselements bildet eine Dosierstellung. Das Aufzieh-Begrenzungselement ist dann axial an dem Dosier-Kolbenkörper fixiert.

Es ist von Vorteil, wenn das Aufzieh-Begrenzungselement auch in seiner äußeren Stellung mit dem Dosier-Kolbenkörper in Rasteingriff steht. Dieser Rasteingriff ermöglicht auch eine Verlagerung des Dosier-Kolbenkörpers in Richtung der Längsmittelachse gegenüber dem Aufzieh-Begrenzungselement. Der Eingriff ist lösbar.

Die Rückmeldung gemäß Anspruch 6 kann beispielsweise taktil und/oder akustisch erfolgen. An dem Dosier-Kolbenkörper sind vorzugsweise mehrere Rückmeldungs-Aussparungen bzw. -Unterbrechungen vorgesehen, die in Richtung der Längsmittelachse beabstandet zueinander angeordnet sind. Bei einer axialen Verschiebung des Dosier-Kolbenkörpers gegenüber dem Aufzieh-Begrenzungselement erfolgt vorzugsweise ein lösbarer Eingriff zwischen dem mindestens einen ersten und/oder zweiten Rastgegenelement und den Aussparungen bzw. Unterbrechungen. Das Aufzieh-Begrenzungselement rattert so vorzugsweise auf dem Dosier-Kolbenkörper bei einer Relativ-Verschiebebewegung zwischen dem Dosier-Kolbenkörper und dem Aufzieh-Begrenzungselement. Das Auffinden der ersten Formschlussmittel wird so somit vorzugsweise akustisch und taktil unterstützt. Das Rattern gibt dem Benutzer also günstigerweise eine taktile und/oder akustische Rückmeldung. Diese äußere Stellung des Aufzieh-Begrenzungselements bildet eine Montagestellung.

Das Betätigungsfenster nach Anspruch 7 ermöglicht eine einfache und sichere Verlagerung des Aufzieh-Begrenzungselements gegenüber dem Dosier - Kolbenkörper.

Bei der Ausgestaltung nach Anspruch 8 bildet das Aufzieh-Begrenzungselement einen Riegel und beschränkt bzw. verhindert so eine Relativbewegung zwischen dem Aufzieh-Begrenzungselement und der Dosierhülse in Richtung der Längsmittelachse. Das Aufzieh-Begrenzungselement schließt außenseitig im Wesentlichen bündig mit der Dosierhülse bzw. deren Hauptzylinder ab. Dies führt zu einem äußerst robusten Dosiersystem. Insbesondere ist so auf einfache Art und Weise verhinderbar, dass das Aufzieh-Begrenzungselement versehentlich in seine innere Stellung verlagert wird, was eine ungewollte Einstellung eines Dosiervolumens zufolge hat.

Bei der Ausgestaltung nach Anspruch 9 ist im Wesentlichen eine Relativbewegung zwischen dem Aufzieh-Begrenzungselement und der Dosierhülse in Richtung der Längsmittelachse unterbunden.

Die Ausgestaltung nach Anspruch 12 führt zu einem Dosiersystem, das äußerst montagefreundlich ist. Der Anschlag ist so im Prinzip einfach bei fortgeschrittener Montage an der Dosierhülse örtlich festlegbar. Der Anschlag kann direkt oder indirekt an der Dosierhülse angeordnet sein. Eine einstückige Ausgestaltung mit der Dosierhülse ist alternativ möglich. Es ist von Vorteil, wenn der Anschlag durch mindestens einen Schnapphaken an der Dosierhülse örtlich festlegbar ist.

Das Dosiersystem nach Anspruch 13 ermöglicht eine besonders große Änderung des einstellbaren Dosiervolumens.

Die Ausgestaltung nach Anspruch 14 führt zu einer äußerst einfachen Einstellung des gewünschten Dosiervolumens.

Die Ausführung nach Anspruch 15 bildet quasi eine Verliersicherung. Das Aufzieh-Begrenzungselement ist unverlierbar mit dem Dosier-Kolbenkörper verbunden. Es kann so nicht verloren gehen.

Es ist von Vorteil, wenn das Dosiersystem bereitgestellt bzw. ausgeliefert wird, wenn sich das Aufzieh-Begrenzungselement in seiner äußeren Stellung bzw. Montagestellung befindet. Es ist dann, zum Beispiel von einem Arzt, Apotheker oder dergleichen, die Vorgabe einer Dosiermenge in einfacher Weise möglich.

In Kombination mit den vorstehenden Ansprüchen bzw. Merkmalen, aber auch als eigenständige Erfindung hat der Dosier-Kolbenkörper an seinem ersten Ende einen in Richtung der Längsmittelachse vorspringenden Verdrängerkörper zum Durchsetzen bzw. Eindringen in die mindestens eine Kommunizierungsöffnung und in seinem Kolbenboden mindestens eine um den Verdrängerkörper laufende Vertiefung, wobei in einem Dosierhülsenboden der Dosierhülse mindestens eine zu der mindestens einen Vertiefung im Wesentlichen komplementäre Erhöhung ausgebildet ist, wobei vorzugsweise der Dosier-Kolbenkörper an seinem Kolbenboden und dem Verdrängerkörper mindestens eine Drainagenut zur Verbesserung der Restentleerung umfasst. Die mindestens eine Drainagenut begrenzt einen Drainagekanal für das fließfähige Medium und ermöglicht ein besonders gutes Abfließen bzw. eine besonders hohe Restentleerung des abzugebenden fließfähigen Mediums. Eine sehr genaue Dosierung des fließfähigen Mediums ist ferner so möglich.

Günstigerweise stehen die mindestens eine Vertiefung und die mindestens eine Erhöhung miteinander in Eingriff, wenn sich der Dosier-Kolbenkörper in seiner vollständig vorgerückten Stellung befindet. Die Vertiefung/en und die Erhöhung/en laufen vorzugsweise ringförmig um die Längsmittelachse.

Die mindestens eine Drainagenut hat beispielsweise einen eckigen oder kreisbogenförmigen Querschnitt.

Es ist von Vorteil, wenn die mindestens eine Drainagenut eine Tiefe hat, die zwischen 0,05 mm und 0,4 mm, bevorzugter zwischen 0,1 mm und 0,25 mm, liegt.

Günstigerweise hat die mindestens eine Drainagenut eine Breite, die zwischen 0,4 mm und 1,2 mm, bevorzugter zwischen 0,6 mm und 1,0 mm, liegt.

Im Bereich des Kolbenbodens verläuft die mindestens eine Drainagenut im Wesentlichen radial zu der Längsmittelachse, während sie sich im Bereich des Verdrängerkörpers im Wesentlichen parallel zu der Längsmittelachse erstreckt. Günstigerweise erstreckt sich jede Drainagenut über ihre Länge in einer Ebene, in der vorzugsweise auch die Längsmittelachse liegt.

Wenn mehrere Drainagenuten vorhanden sind, so haben diese günstigerweise einen identischen angularen Abstand zueinander.

Es ist von Vorteil, wenn sich die mindestens eine Drainagenut in dem Kolbenboden von der Längsmittelachse ausgehend nach radial außen verbreitert, bevorzugter im Wesentlichen kontinuierlich nach radial außen verbreitert.

Nachfolgend wird unter Bezugnahme auf die beigefügte Zeichnung eine bevorzugte Ausführungsform der Erfindung beispielhaft beschrieben. Dabei zeigen:
- Fig. 1: eine Seitenansicht eines erfindungsgemäßen Dosiersystems, bei der sich das Aufzieh-Begrenzungselement in seiner äußeren Stellung befindet,
- Fig. 2: einen Querschnitt durch das erfindungsgemäße Dosiersystem entsprechend der Schnittlinie II-II in Fig. 1,
- Fig. 3: eine Seitenansicht des erfindungsgemäßen Dosiersystems entsprechend Fig. 1, wobei sich das AufziehBegrenzungselement in seiner inneren Stellung befindet,
- Fig. 4: einen Querschnitt durch das erfindungsgemäße Dosiersystem entsprechend der Schnittlinie IV-IV in Fig. 3,
- Fig. 5: einen ersten Längsschnitt durch das in den Fig. 1 bis 4 dargestellte erfindungsgemäße Dosiersystem,
- Fig. 6: einen zweiten Längsschnitt durch das in den Fig. 1 bis 5 dargestellte Dosiersystem, wobei hier der Längsschnitt verglichen mit Fig. 5 um die Längsmittelachse verkippt ist, und

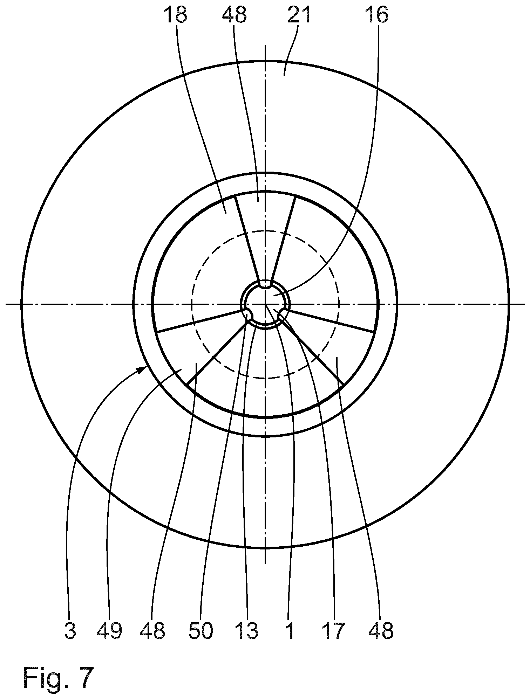

- Fig. 7: eine Ansicht, die das in den Figuren 1 bis 6 veranschaulichte Dosiersystem von unten zeigt.

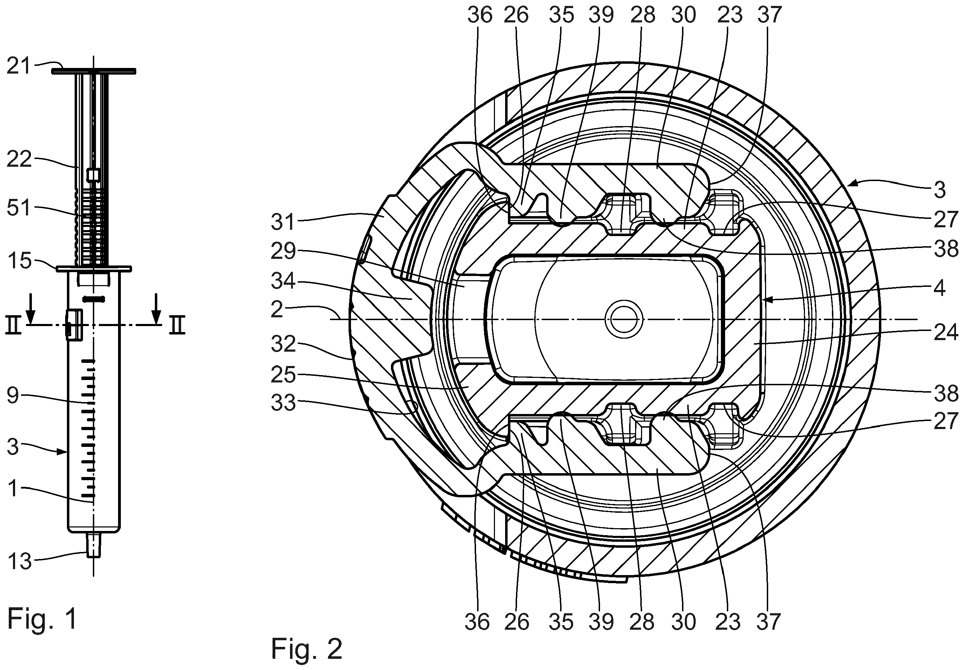

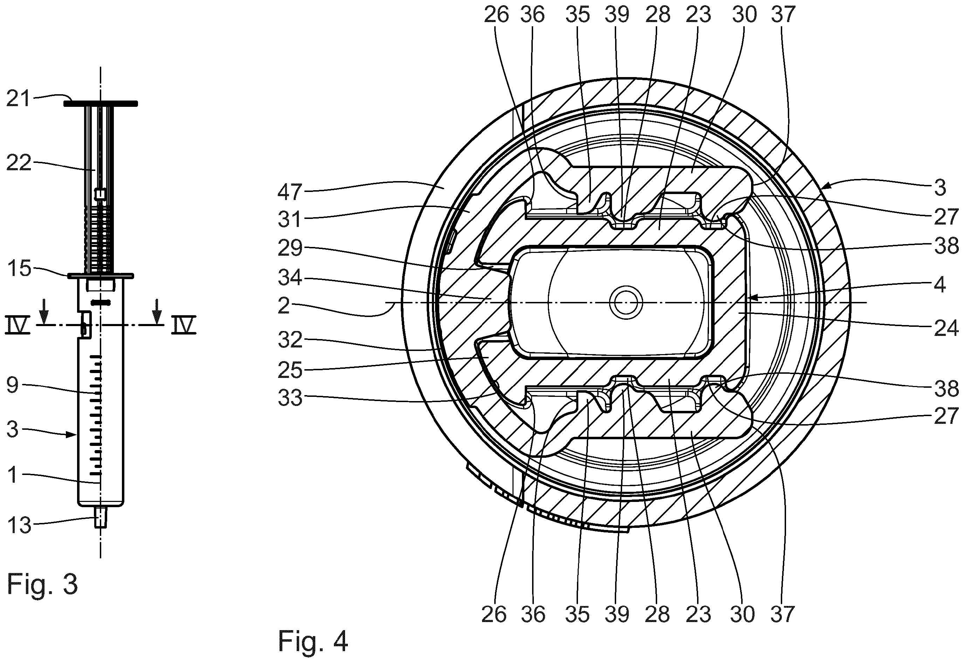

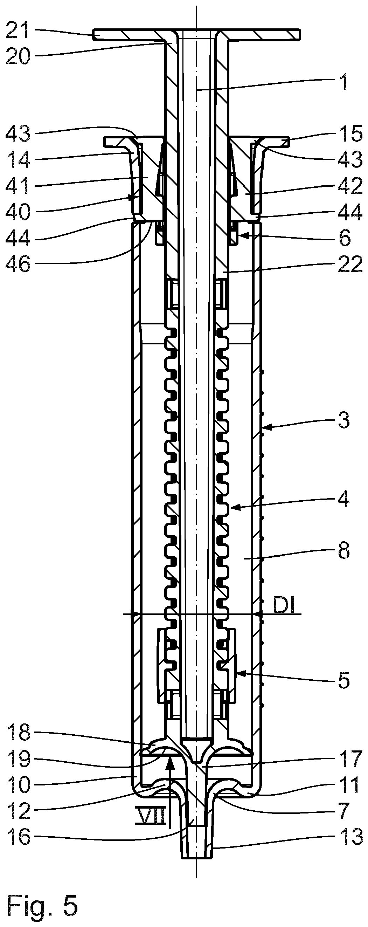

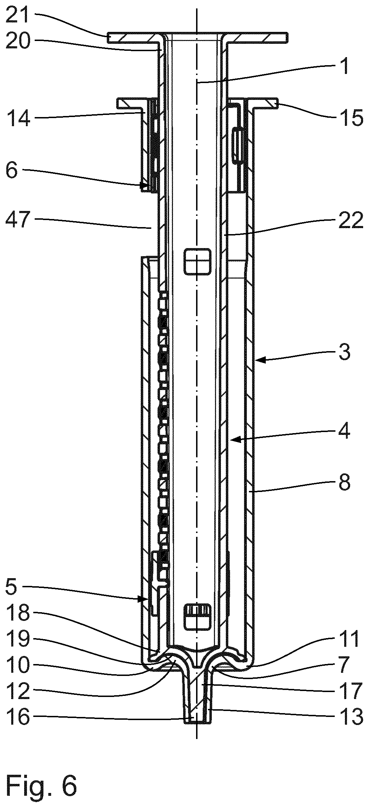

Ein in den Fig. 1 bis 6 dargestelltes Dosiersystem hat eine Längsmittelachse 1 und ist bezüglich einer Symmetrieebene 2 symmetrisch ausgeführt. Die Längsmittelachse 1 liegt in der Symmetrieebene 2.

Das Dosiersystem umfasst eine Dosierhülse 3 und einen in der Dosierhülse 3 relativ zu dieser in Richtung der Längsmittelachse 1 verschiebbar geführten Dosier-Kolbenköper 4. Der Dosier-Kolbenkörper 4 ist zwischen einer aufgezogenen Stellung und einer vorgerückten Stellung axial gegenüber der Dosierhülse 3 verschiebbar. Das Dosiersystem hat außerdem ein Aufzieh-Begrenzungselement 5, das an dem Dosier-Kolbenkörper 4 in verschiedenen, in Richtung der Längsmittelachse 1 voneinander beabstandeten Positionen axial festlegbar ist und so den maximalen Aufziehweg des Dosier-Kolbenkörpers 4 örtlich begrenzt. An der Dosierhülse 3 ist zur mechanischen Wechselwirkung mit dem Aufzieh-Begrenzungselement 5 in der aufgezogenen Stellung ein Anschlag 6 angeordnet. Die Dosierhülse 3 hat eine Kommunizierungsöffnung 7 zur Kommunizierung mit der Umgebung. Sie begrenzt einen Aufbewahrungsraum 8 für ein fließfähiges Medium (nicht dargestellt).

In Abhängigkeit der axialen örtlichen Festlegung des Aufzieh-Begrenzungselements 5 gegenüber dem Dosier-Kolbenkörper 4 ist der maximale Aufziehweg des Dosier-Kolbenkörpers 4 bzw. das maximale Dosiervolumen individuell einstellbar. Der maximale Aufziehweg ist durch den Anschlag 6 und das Aufzieh-Begrenzungselement 5 begrenzt.

Wenn der Dosier-Kolbenkörper 4 von seiner vorgerückten Stellung in eine aufgezogene Stellung axial überführt wird, kann ein flüssiges Medium über die Kommunizierungsöffnung 7 in den Aufbewahrungsraum 8 eingesaugt werden. Wenn dagegen der Dosier-Kolbenkörper 4 von einer aufgezogenen Stellung in seine vorgerückte Stellung axial überführt wird, kann dagegen das in dem Aufbewahrungsraum 8 befindliche Medium über die Kommunizierungsöffnung 7 abgegeben bzw. hinaus gespritzt werden. In der aufgezogenen Stellung ist der Dosier-Kolbenkörper 4 aus der Dosierhülse 3 zumindest teilweise herausgezogen, während in der vorgerückten Stellung der Dosier-Kolbenkörper 4 ganz in die Dosierhülse 3 eingeschoben ist.

Die Dosierhülse 3 ist aus Kunststoff gebildet, der vorzugsweise transparent ist. Sie hat eine Dosierskalierung 9. Die Dosierhülse 3 ist als Zylinderkörper ausgeführt, der im Wesentlichen einen konstanten Innendurchmesser DI in Richtung der Längmittelachse 1 hat und an einem ersten Ende 10 durch einen Dosierhülsenboden 11 verschlossen ist. Der Dosierhülsenboden 11 hat hier eine nach innen in den Aufbewahrungsraum 8 springende, ringartige Wulst 12, die um die Längsmittelachse 1 läuft. In dem Dosierhülsenboden 11 ist die Kommunizierungsöffnung 7 mittig angeordnet. An den Dosierhülsenboden 11 schließt sich im Bereich der Kommunizierungsöffnung 7 außen ein Rohrstück 13 an, dessen Innendurchmesser wesentlich kleiner als der Innendurchmesser DI der Dosierhülse 3 ist.

Die Dosierhülse 3 hat außerdem ein zweites Ende 14, das gegenüberliegend zu dem ersten Ende 10 ist. Im Bereich des zweiten Endes 14 springt ein Umfangsbund 15 radial nach außen vor, der einen Griff bildet.

Der Dosier-Kolbenkörper 4 ist ebenfalls aus Kunststoff gebildet. Er hat an seinem ersten Ende 16 einen in Richtung der Längsmittelachse 1 vorspringenden Verdrängerkörper bzw. Ausstoßkörper 17, dessen Außendurchmesser im Wesentlichen entsprechend dem Innendurchmesser des Rohrstücks 13 ist. Die axiale Länge des Verdrängerkörpers 17 entspricht im Wesentlichen der axialen Länge des Rohrstücks 13. Der Verdrängerkörper 17 ist im Wesentlichen zylindrisch ausgeführt und hat somit einen kreisförmigen, geschlossenen Querschnitt.

An den Verdrängerkörper 17 schließt sich ein radial vorspringender Kolbenboden 18 an, der Bestandteil des Dosier-Kolbenkörpers 4 ist und einen Außendurchmesser aufweist, der im Wesentlichen dem Innendurchmesser DI der Dosierhülse 3 entspricht. Der Kolbenboden 18 liegt randseitig dicht innen an der Dosierhülse 3 an. In dem Kolbenboden 18 ist an der dem Dosierhülsenboden 11 zugewandten Seite eine ringförmige Vertiefung 19 vorgesehen, die sich um die Längsmittelachse 1 erstreckt und komplementär zu der Wulst 12 ausgeführt ist.

Der Dosier-Kolbenkörper 4 hat drei zueinander beabstandet angeordnete Drainagenuten 48, die sich in dem Kolbenboden 18 an der dem Dosierhülsenboden 11 zugewandten Seite und in dem Verdrängerkörper 17 erstrecken. Jede Drainagenut 48 hat somit in dem Kolbenboden 18 einen Drainagenut-Bodenabschnitt 49 und einen sich an diesen direkt anschließenden Drainagenut-Verdrängerkörperabschnitt 50 in dem Verdrängerkörper 17, die miteinander in direkter Drainage- bzw. Strömungsverbindung stehen.

Die Drainagenut-Bodenabschnitte 49 gehen jeweils von dem Außenrand des Kolbenbodens 18 aus und verlaufen von diesem radial nach innen in Richtung auf die Längsmittelachse 1 bis zu dem Verdrängerkörper 17. Sie verjüngen sich dabei kontinuierlich.

Die Drainagenut-Verdrängerkörperabschnitte 50, die mit den jeweiligen Drainagenut-Bodenabschnitten 49 in Drainage- bzw. Strömungsverbindung stehen, erstrecken sich in dem Verdrängerkörper 17 von dem zu dem Kolbenboden 18 unmittelbar benachbarten Bereich bis zu dessen freien Ende. Sie haben jeweils eine im Wesentlichen gleichbleibende Breite und erstrecken sich im Wesentlichen parallel zu der Längsmittelachse 1.

Jede Drainagenut 48 liegt in einer Ebene, die auch die Längsmittelachse 1 einschließt. Eine andere Anzahl von Drainagenuten 48 ist alternativ möglich. Die Drainagenuten 48 sind unabhängig voneinander.

Gegenüberliegend zu dem ersten Ende 16 hat der Dosier-Kolbenkörper 4 ein zweites Ende 20, an dem ein radial nach außen vorspringender Betätigungsvorsprung 21 angeordnet ist.

Zwischen dem Kolbenboden 18 und dem zweiten Betätigungsvorsprung 21 hat der Dosier-Kolbenkörper 4 einen Schaftbereich 22. In dem Schaftbereich 22 hat der Dosier-Kolbenkörper 4 im Wesentlichen einen rechteckigen Querschnitt. Der Dosier-Kolbenkörper 4 ist dort im Wesentlichen als Vierkant-Profilkörper ausgeführt. Er weist in dem Schaftbereich 22 zwei einander gegenüberliegende Seitenwände 23 auf, die parallel zueinander verlaufen und über eine Innenwand 24 und eine vordere Wand 25 miteinander verbunden sind. Die Seitenwände 23 erstrecken sich parallel zu der Symmetrieebene 2, während die Innenwand 24 und die vordere Wand 25 im Wesentlichen senkrecht zu dieser verlaufen. Die Quererstreckung des Dosier-Kolbenkörpers 4 in seinem Schaftbereich 22 ist kleiner als der Innendurchmesser DI der Dosierhülse 3.

Die vordere Wand 25 steht unter Bildung von hinteren Rückhalteschultern 26 seitlich gegenüber den Seitenwänden 23 vor. Sie ist außerdem zwischen den Seitenwänden 23 leicht nach außen gekrümmt.

In jeder Seitenwand 23 sind hier eine Vielzahl erster und zweiter Rastelemente 27 bzw. 28 außenseitig angeordnet, die als Rastelementaufnahmen ausgebildet sind und nach seitlich außen offen sind. Die ersten und zweiten Rastelementaufnahmen 27, 28 sind jeweils separat ausgeführt und senkrecht zu der Längsmittelachse 1 beabstandet zueinander angeordnet. Die in den Seitenwänden 23 angeordneten ersten Rastelementaufnahmen 27 liegen einander gegenüber und haben einen identischen Abstand zu der vorderen Wand 25. Die in den Seitenwänden 23 angeordneten zweiten Rastelementaufnahmen 28 liegen wieder einander gegenüber und haben einen identischen Abstand zu der vorderen Wand 25. Die ersten Rastelementaufnahmen 27 sind weiter von der vorderen Wand 25 als die zweiten Rastelementaufnahmen 28 angeordnet. Eine andere Anzahl von bezogen auf die vordere Wand 25 hintereinander angeordneten Rastelementaufnahmen 27, 28 ist alternativ möglich.

Die in den Seitenwänden 23 angeordneten ersten Rastelementaufnahmen 27 sind in Richtung der Längmittelachse 1 beabstandet zueinander in einer ersten Reihe angeordnet. Die in den Seitenwänden 23 angeordneten zweiten Rastelementaufnahmen 28 sind in Richtung der Längmittelachse 1 beabstandet zueinander in einer zweiten Reihe angeordnet. Die ersten und zweiten Rastelementaufnahmen 27 bzw. 28 sind hier als Vertiefungen ausgeführt. Eine andere Ausgestaltung ist alternativ möglich.

Ferner ist in der vorderen Wand 25 mittig eine Vielzahl von separaten Formschlussaufnahmen 29 außenseitig bzw. vorne angeordnet. Auch die Formschlussaufnahmen 29 in der vorderen Wand 25 sind in Richtung der Längmittelachse 1 beabstandet zueinander in einer Reihe angeordnet. Die Formschlussaufnahmen 29 sind dagegen als Durchbrechungen ausgeführt, die vorzugsweise eine längliche, rechteckige Form haben und parallel zueinander verlaufen.

Es ist von Vorteil, wenn die einander gegenüberliegenden ersten und zweiten Rastelementaufnahmen 27 bzw. 28 und eine benachbarte Formschlussaufnahme 29 jeweils in einer gemeinsamen Ebene liegen.

Günstigerweise sind die ersten und zweiten Rastelementaufnahmen 27, 28 sowie die Formschlussaufnahme 29 über einen Bereich an dem Dosier-Kolbenkörper 4 vorgesehen, der sich über mindestens 30 % der Gesamtlänge des Dosier-Kolbenkörpers 4 erstreckt.

In jeder Seitenwand 23 sind mehrere Rückmeldungs-Aussparungen bzw. - Unterbrechungen 51 vorgesehen, die jeweils in Richtung der Längsmittelachse 1 beabstandet zueinander angeordnet sind und sich jeweils über die gesamte Breite der jeweiligen Seitenwand 23 erstrecken. Die Rückmeldungs-Aussparungen 51 erstrecken sich jeweils im Wesentlichen senkrecht zwischen der Innenwand 24 und der vorderen Wand 25.

Das Aufzieh-Begrenzungselement 5 ist aus Kunststoff gebildet. Es ist klammerartig ausgeführt und hat zwei einander gegenüberliegende U-Schenkel 30, die im Wesentlichen parallel zueinander verlaufen und über einen U-Boden 31 miteinander verbunden sind. Es ist von Vorteil, wenn die U-Schenkel 30 eine Federbewegung aufeinander zu und voneinander weg ausführen können.

Der U-Boden 31 hat eine nach außen gewandte Außenseite 32, deren Krümmung an die Innenkrümmung der Dosierhülse 3 angepasst ist. Gegenüberliegend zu der Außenseite 32 hat der U-Boden 31 eine Innenseite 33, deren Krümmung an die Krümmung der vorderen Wand 25 angepasst ist. Von der Innenseite 33 springt mittig ein Formschlussmittel 34 in Form eines Vorsprungs senkrecht nach innen vor. Der Querschnitt des Formschlussmittels 34 ist an den Querschnitt der Formschlussaufnahmen 29 angepasst. Es ist von Vorteil, wenn sich das Formschlussmittel 34 von der Innenseite 33 des U-Bodens 31 aus verjüngt.

Benachbart zu dem U-Boden 31 ist an den beiden U-Schenkeln 30 innenseitig jeweils ein Rückhaltevorsprung 35 mit einer entsprechenden, dem U-Boden 31 zugewandten Rückhaltefläche 36 angeordnet.

Gegenüberliegend zu dem U-Boden 31 hat jeder U-Schenkel 30 ein inneres Ende 37. An jedem U-Schenkel 30 ist innenseitig im Bereich des inneren Endes 37 ein erstes, vorspringendes Rastgegenelement 38 angeordnet. Die ersten Rastgegenelemente 38 springen von den U-Schenkeln 30 aufeinander zu. Die ersten Rastgegenelemente 38 sind in ihrer Form an die ersten Rastelementaufnahmen 27 angepasst.

Ferner ist an jedem U-Schenkel 30 ein zweites, vorspringendes Rastgegenelement 39 innenseitig angeordnet, das zwischen dem U-Boden 31 und dem ersten Rastgegenelement 38 angeordnet ist. Die zweiten Rastgegenelemente 39 springen von den U-Schenkeln 30 aufeinander zu. Sie sind in ihrer Form an die zweiten Rastelementaufnahmen 28 angepasst.

Der Abstand der ersten und zweiten Rastgegenelemente 38 bzw. 39 an einem U-Schenkel 30 zueinander entspricht im Wesentlichen dem Abstand der ersten und zweiten Rastelementaufnahmen 27 bzw. 28 an einer Seitenwand 23. Es ist von Vorteil, wenn die ersten und zweiten Rastgegenelemente 38 bzw. 39 und das Formschlussmittel 34 in einer gemeinsamen Ebene liegen. Eine versetzte Anordnung ist alternativ möglich.

Das Aufzieh-Begrenzungselement 5 ist in seinen Querabmessungen senkrecht zur der Längsmittelachse 1 kleiner als der Innendurchmesser DI der Dosierhülse 3. Dies betrifft sowohl Breite als auch Tiefe. Das Aufzieh-Begrenzungselement 5 ist so in der Dosierhülse 3 unterbringbar.

Im Bereich des zweiten Endes 14 ist in die Dosierhülse 3 ein Verschlusskörper 40 eingesetzt, der buchsenartig ausgeführt ist. Der Verschlusskörper 40 ist durch zwei Verschlusskörperhälften 41, 42 gebildet, die über ein Scharnier, vorzugsweise ein Filmscharnier, gelenkig miteinander verbunden sind. Die Scharnierachse erstreckt sich parallel zu der Längsmittelachse 1. Der Dosier-Kolbenkörper 4 durchsetzt den Verschlusskörper 40 mit seinem Schaftbereich 22.

Gegenüberliegend zu dem Scharnier ist an der einen Verschlusskörperhälfte 41 eine Rastnase angeordnet, die im zusammengeklappten Zustand der Verschlusskörperhälften 41, 42 mit einer Rastausnehmung an der anderen Verschlusskörperhälfte 42 in rastendem Eingriff steht und so die Verschlusskörperhälften 41, 42 im zusammengeklappten Zustand aneinander festlegt.

An den Verschlusskörperhälften 41, 42 ist oben außenseitig ein radial vorspringender Anlagevorsprung 43 vorgesehen, der im Bereich des Umfangbunds 15 an der Dosierhülse 3 oben anliegt. Ferner hat jede Verschlusskörperhälfte 41, 42 unten einen radial vorspringenden Rastkörper 44, der von innen in eine entsprechend dimensionierte und platzierte Rastausnehmung 45 an der Dosierhülse 3 eingreift. Durch die Anlagevorsprünge 43 und die Rastkörper 44 ist der Verschlusskörper 40 an der Dosierhülse 3 axial und angular fixiert und begrenzt diese.

Alternativ ist im Bereich des zweiten Endes 14 in die Dosierhülse 3 der Verschlusskörper 40, der beispielsweise die Form einer Buchse hat, eingeschraubt, eingeklebt, eingesteckt oder dergleichen. Der Verschlusskörper 40 ist dann entsprechend ausgebildet.

Bei einer nicht dargestellten Variante des Dosiersystems ist der Verschlusskörper 40 nicht als separates Bauteil ausgebildet. Ein die Funktion des Verschlusskörpers übernehmendes Bauteil kann als Konstruktionselement in einen beispielsweise mit Filmscharnieren abgetrennten Bereich des Dosier-Kolbenkörpers 4 integriert sein. Bei der Montage des Dosiersystems können diese Konstruktionselemente dann beispielsweise durch Umklappen als Anschlag für das Aufzieh-Begrenzungselement 5 wirken.

Der Verschlusskörper 40 hat außerdem eine innere, senkrecht zu der Längsmittelachse 1 orientierte Bodenfläche 46, von der der Anschlag 6 in Richtung der Längsmittelachse 1 nach unten in Richtung auf den Dosierhülsenboden 11 vorspringt. Der Anschlag 6 ist im Wesentlichen ringförmig ausgeführt und erstreckt sich um die Längsmittelachse 1. Er umläuft den Dosier-Kolbenkörper 4 bzw. dessen Schaftbereich 22.

Im Bereich des Anschlags 6 ist in der Dosierhülse 3 ein Betätigungsfenster 47 angeordnet, das sich über einen Umfangsbereich der Dosierhülse 3 erstreckt und einen Zugang von außen zu dem Aufnahmeraum 8 gewährt. Das Betätigungsfenster 47 hat in Richtung der Längsmittelachse 1 eine Erstreckung, die im Wesentlichen der Erstreckung des Aufzieh-Begrenzungselements 5 in Richtung der Längsmittelachse 1 entspricht. Ferner hat das Betätigungsfenster 47 eine Breite senkrecht zu der Längsmittelachse 1, die breiter als die Breite des Aufzieh-Begrenzungselements 5 im Bereich des U-Bodens 31 senkrecht zu der Längsmittelachse 1 ist.

Nachfolgend wird das Dosiersystem im Einsatz näher beschrieben. Dabei wird von der in Fig. 1 gezeigten Stellung ausgegangen. Der Kolbenboden 18 ist dabei beabstandet zu dem Dosierhülsenboden 11 angeordnet. Der Dosier-Kolbenkörper 4 befindet sich in einer aufgezogenen Stellung. Das Aufzieh-Begrenzungselement 5 ist in seiner radial äußeren Stellung, sodass der U-Boden 31 des Aufzieh-Begrenzungselements 5 das Betätigungsfenster 47 von innen nach außen riegelartig durchsetzt und so benachbart zu den das Betätigungsfenster 47 begrenzenden Wänden der Dosierhülse 3 angeordnet ist. Der U-Boden 31 ist beabstandet zu der vorderen Wand 25 angeordnet. Die ersten und zweiten Rastgegenelemente 38 bzw. 39 stehen außer Eingriff mit den ersten bzw. zweiten Rastelementaufnahmen 27 bzw. 28. Das Formschlussmittel 34 an dem Aufzieh-Begrenzungselement 5 steht außerdem außer Eingriff mit den Formschlussaufnahmen 29. Der Dosier-Kolbenkörper 4 ist gegenüber der Dosierhülse 3 bzw. gegenüber dem Aufzieh-Begrenzungselement 5 in Richtung der Längsmittelachse 1 verlagerbar. Die Rückhalteflächen 36 des Aufzieh-Begrenzungselements 5 liegen innenseitig an den Rückhalteschultern 26 des Dosier-Kolbenkörpers 4 an, sodass ein weiteres radiales Herausziehen des Aufzieh-Begrenzungselements 5 unterbunden ist.

Der Dosier-Kolbenkörper 4 wird nun händisch in seine gewünschte Dosierstellung entlang der Längsmittelachse 1 bewegt. Eine entsprechende Kraft kann dabei zwischen dem Betätigungsvorsprung 21 und dem Umfangsbund 15 ausgeübt werden. Es erfolgt eine axiale Relativbewegung zwischen dem Dosier-Kolbenkörper 4 und der Dosierhülse 3 und dem Aufzieh-Begrenzungselement 5. Dabei gibt es eine akustische und taktile Rückmeldung für den Benutzer, die auf den zeitweisen Eingriff der ersten und zweiten Rastgegenelemente 38, 39 in die Rückmeldungs-Aussparungen 51 zurückzuführen ist. Wenn die gewünschte Dosierstellung des Dosier-Kolbenkörpers 4 gegenüber der Dosierhülse 3 erreicht ist, wird das Aufzieh-Begrenzungselement 5 über seinen Betätigungskopf in radialer Richtung von seiner äußeren Stellung in seine innere Stellung gedrückt, wobei vorzugsweise eine Reibkraft zwischen dem Aufzieh-Begrenzungselement 5 und dem Dosier-Kolbenkörper 4 und/oder der Dosierhülse 3 überwunden werden muss. Beim radialen Verlagern des Aufzieh-Begrenzungselements 5 nach innen wird das Formschlussmittel 34 in die benachbarte Formschlussaufnahme 29 von vorne bewegt, während die ersten und zweiten Rastgegenelemente 38 bzw. 39 in die benachbarten ersten und zweiten Rastelementaufnahmen 27 bzw. 28 seitlich einrasten und so das Aufzieh-Begrenzungselement 5 unlösbar an dem Dosier-Kolbenkörper 4 axial und radial festlegen. Der U-Boden 31 befindet sich nun auch vollständig innerhalb der Dosierhülse 3 bzw. in deren Aufbewahrungsraum 8. Er ist nun nach innen beabstandet zu den das Betätigungsfenster 47 begrenzenden Wänden der Dosierhülse 3 angeordnet. Der U-Boden 31 liegt außen an der vorderen Wand 25 an.

Nun kann der Dosier-Kolbenkörper 4 soweit aufgezogen werden, bis das Aufzieh-Begrenzungselement 5 oben an dem Anschlag 6 anschlägt. Wenn der Dosier-Kolbenkörper 4 in seine vorgerückte Stellung geschoben wird, wird das in dem Aufnahmeraum 8 befindliche Medium über die Kommunizierungsöffnung 7 ausgestoßen. Bei der Verlagerung des Dosier-Kolbenkörpers 4 bewegt sich das Aufzieh-Begrenzungselement 5 mit dem Dosier-Kolbenkörper 4 mit. Die Außenseite 32 bewegt sich in der inneren Stellung des Aufzieh-Begrenzungselements 5 innerhalb der Dosierhülse 3 axial gegenüber derselben. Die inneren Enden 37 sind in der inneren Stellung des Aufzieh-Begrenzungselements 5 immer noch beabstandet zu der Innenseite der Dosierhülse 3 angeordnet.

In der vollständig vorgerückten Stellung greift die Wulst 12 in die Vertiefung 19, sodass eine besonders gute Entleerung des in dem Aufnahmeraum 8 befindlichen Mediums möglich ist. Der Verdrängerkörper 17 greift in das Rohrstück 13 ein und drängt das in diesem sich befindende zu verabreichende Medium durch das Rohrstück 13 nach außen. Der Verdrängerkörper 17 wirkt also als zusätzlicher Verdränger, so dass das zu verabreichende Medium im Wesentlichen ohne verbleibenden Rest in der Aufnahme 8 verabreichbar ist. Das zu verabreichende Medium gelangt über die Drainagenut/en 48 aus dem Aufnahmeraum 8, wenn der Verdrängerkörper 17 in das Rohrstück 13 eingreift. Die Drainagenut/en 48 begünstigt/en das Restentleeren.

## Patentansprüche

1. Dosiersystem zur Einstellung eines Dosiervolumens eines fließfähigen Mediums, umfassend
a) eine Längsmittelachse (1),
b) eine Dosierhülse (3) mit mindestens einer Kommunizierungsöffnung (7) zur Kommunizierung mit der Umgebung, und
c) einen Dosier-Kolbenkörper (4), der in der Dosierhülse (3) in der Richtung der Längsmittelachse (1) zur Aufnahme und Abgabe des Mediums über die mindestens eine Kommunizierungsöffnung (7) zwischen einer aufgezogenen Stellung und einer vorgerückten Stellung verschiebbar geführt ist,
**gekennzeichnet durch**
d) ein Aufzieh-Begrenzungselement (5) zum Begrenzen eines maximalen Aufziehwegs des Dosier-Kolbenkörpers (4) in der Richtung der Längsmittelachse (1),
i) wobei das Aufzieh-Begrenzungselement (5) in mindestens einer Position an dem Dosier-Kolbenkörper (4) axial festlegbar ist,
ii) wobei das Aufzieh-Begrenzungselement (5) radial zu der Längsmittelachse (1) zwischen einer radial inneren, axial an dem Dosier-Kolbenkörper (4) festgelegten Stellung und einer radial äußeren Stellung gegenüber dem Dosier-Kolbenkörper (4) verlagerbar ist, und
iii) wobei in der radial äußeren Stellung des Aufzieh-Begrenzungselements (5) der Dosier-Kolbenkörper (4) gegenüber der Dosierhülse (3) und dem Aufzieh-Begrenzungselement (5) in Richtung der Längsmittelachse (1) verlagerbar ist, und
iv) wobei das Aufzieh-Begrenzungselement (5) in der inneren Stellung vollständig innerhalb der Dosierhülse (3) angeordnet ist, und
e) einen an der Dosierhülse (3) angeordneten Anschlag (6) zum Wechselwirken mit dem Aufzieh-Begrenzungselement (5) in der aufgezogenen Stellung des Dosier-Kolbenkörpers (4).

2. Dosiersystem nach Anspruch 1, **dadurch gekennzeichnet, dass** eine maximale Erstreckung des Aufzieh-Begrenzungselements (5) senkrecht zu der Längmittelachse (1) kleiner ist als ein Innendurchmesser DI der Dosierhülse (3).

3. Dosiersystem nach einem der vorherigen Ansprüche, **dadurch gekennzeichnet, dass** das Aufzieh-Begrenzungselement (5) in der inneren Stellung durch formschlüssige Verbindung, vorzugsweise durch eine Rastverbindung, an dem Dosier-Kolbenkörper (4) örtlich axial festgelegt ist.

4. Dosiersystem nach Anspruch 3, **dadurch gekennzeichnet, dass** an dem Dosier-Kolbenkörper (4) mehrere erste Formschlussmittel (29) vorgesehen sind, die in der Richtung der Längsmittelachse (1) beabstandet zueinander angeordnet sind, wobei an dem Aufzieh-Begrenzungselement (5) mindestens ein zweites Formschlussmittel (34) zur formschlüssigen Wechselwirkung mit mindestens einem der ersten Formschlussmittel (29) vorgesehen ist.

5. Dosiersystem nach einem der vorherigen Ansprüche, **dadurch gekennzeichnet, dass** das Aufzieh-Begrenzungselement (5) in der inneren Stellung unlösbar an dem Dosier-Kolbenkörper (4) örtlich festgelegt ist.

6. Dosiersystem nach einem der vorherigen Ansprüche, **dadurch gekennzeichnet, dass** das sich in der äußeren Stellung befindende Aufzieh-Begrenzungselement (5) beim axialen Verschieben des Dosier-Kolbenkörpers (4) zum Geben einer Rückmeldung in Bezug auf die mindestens eine Position zum axialen Festlegen des Aufzieh-Begrenzungselements (5) an dem Dosier-Kolbenkörper (4) mit dem Dosier-Kolbenkörper (4) wechselwirkt.

7. Dosiersystem nach einem der vorherigen Ansprüche, **dadurch gekennzeichnet, dass** in der Dosierhülse (3) seitlich ein Betätigungsfenster (47) für das Aufzieh-Begrenzungselement (5) vorgesehen ist.

8. Dosiersystem nach Anspruch 7, **dadurch gekennzeichnet, dass** das Aufzieh-Begrenzungselement (5) in der äußeren Stellung das Betätigungsfenster (47) riegelartig durchsetzt und vorzugsweise außen im Wesentlichen bündig mit der Dosierhülse (3) abschließt.

9. Dosiersystem nach Anspruch 7 oder 8, **dadurch gekennzeichnet, dass** das Aufzieh-Begrenzungselement (5) in Richtung der Längsmittelachse (1) eine Erstreckung aufweist, die im Wesentlichen der Erstreckung des Betätigungsfensters (47) in Richtung der Längsmittelachse (1) entspricht.

10. Dosiersystem nach einem der Ansprüche 7 bis 9, **dadurch gekennzeichnet, dass** das Aufzieh-Begrenzungselement (5) senkrecht zu der Längsmittelachse (1) eine Breite aufweist, die im Wesentlichen der Breite des Betätigungsfensters (47) senkrecht zu der Längsmittelachse (1) entspricht.

11. Dosiersystem nach einem der Ansprüche 7 bis 10, **dadurch gekennzeichnet, dass** das Betätigungsfenster (47) benachbart zu dem Anschlag (6), vorzugsweise in Aufziehrichtung des Dosier-Kolbenkörpers (4) unmittelbar vor dem Anschlag (6), angeordnet ist.

12. Dosiersystem nach einem der vorherigen Ansprüche, **dadurch gekennzeichnet, dass** der Anschlag (6) separat zu der Dosierhülse (3) ausgebildet ist und vorzugsweise durch Formschluss, bevorzugter durch Rastverbindung, an der Dosierhülse (3) befestigt ist.

13. Dosiersystem nach einem der vorherigen Ansprüche, **dadurch gekennzeichnet, dass** der Anschlag (6) benachbart zu einem zweiten Ende (20) der Dosierhülse (3) angeordnet ist, das gegenüberliegend zu der mindestens einen Kommunizierungsöffnung (7) ist.

14. Dosiersystem nach einem der vorherigen Ansprüche, **dadurch gekennzeichnet, dass** der Dosier-Kolbenkörper (4) gegenüber dem Aufzieh-Begrenzungselement (5) axial verlagerbar ist, wenn sich das Aufzieh-Begrenzungselement (5) in der äußeren Stellung befindet.

15. Dosiersystem nach einem der vorherigen Ansprüche, **gekennzeichnet durch** eine zwischen dem Dosier-Kolbenkörper (4) und dem Aufzieh-Begrenzungselement (5) wirkende Rückhalteeinrichtung, die die radiale Verlagerung des Aufzieh-Begrenzungselements (5) nach außen begrenzt.

## Claims

1. Dosing system for adjusting a dose volume of a fluid medium, comprising
a) a longitudinal center axis (1),
b) a dosing sleeve (3) with at least one communication opening (7) for communicating with the surroundings,
c) a dosing plunger (4), which can be displaced in the dosing sleeve (3) in the direction of the longitudinal center axis (1) between a retracted position and a depressed position in order to take up and dispense the medium through the at least one communication opening (7),
**characterized by**
d) a retraction limiting element (5) to delimit a maximum retraction path of the dosing plunger (4) in the direction of the longitudinal center axis (1),
i) wherein the retraction limiting element (5) can be axially fixed in at least one position to the dosing plunger (4),
ii) wherein the retraction limiting element (5) can be displaced radially to the longitudinal center axis (1) between a radially inner position, axially fixed to the dosing plunger (4), and a radially outer position in relation to the dosing plunger (4), and
iii) wherein in the radially outer position of the retraction limiting element (5), the dosing plunger (4) can be displaced in relation to the dosing sleeve (3) and the retraction limiting element (5) in the direction of the longitudinal center axis (1), and
iv) wherein the retraction limiting element (5) in the inner position is entirely arranged inside the dosing sleeve (3), and
e) an end stop (6) arranged on the dosing sleeve (3) for interacting with the retraction limiting element (5) in the retracted position of the dosing plunger (4).

2. Dosing system according to claim 1, **characterized in that** a maximum dimension of the retraction limiting element (5) perpendicular to the longitudinal center axis (1) is smaller than an inner diameter DI of the dosing sleeve (3).

3. Dosing system according to one of the preceding claims, **characterized in that** the retraction limiting element (5) in the inner position is locally axially fixed by form-fitting connection, preferably by a locking connection, to the dosing plunger (4).

4. Dosing system according to claim 3, **characterized in that** several first form-fitting means (29) are provided on the dosing plunger (4), being arranged at a spacing from each other in the direction of the longitudinal center axis (1), while at least one second form-fitting means (34) is provided on the retraction limiting element (5) for the form-fitting interaction with at least one of the first form-fitting means (29).

5. Dosing system according to one of the preceding claims, **characterized in that** the retraction limiting element (5) in the inner position is inseparably locally fixed to the dosing plunger (4).

6. Dosing system according to one of the preceding claims, **characterized in that** the retraction limiting element (5) situated in the outer position interacts with the dosing plunger (4) upon axial displacement of the dosing plunger (4) in order to give a feedback in relation to the at least one position for the axial fixation of the retraction limiting element (5) to the dosing plunger (4).

7. Dosing system according to one of the preceding claims, **characterized in that** an activating window (47) for the retraction limiting element (5) is provided in the dosing sleeve (3) at the side.

8. Dosing system according to claim 7, **characterized in that** the retraction limiting element (5) in the outer position passes through the activating window (47) like a bar and is preferably flush on the outside with the dosing sleeve (3).

9. Dosing system according to claim 7 or 8, **characterized in that** the retraction limiting element (5) has a dimension in the direction of the longitudinal center axis (1) that basically corresponds to the dimension of the activating window (47) in the direction of the longitudinal center axis (1).

10. Dosing system according to one of claims 7 to 9, **characterized in that** the retraction limiting element (5) has a width perpendicular to the longitudinal center axis (1) which essentially corresponds to the width of the activation window (47) perpendicular to the longitudinal center axis (1).

11. Dosing system according to one of claims 7 to 10, **characterized in that** the activation window (47) is arranged adjacent to the end stop (6), preferably immediately in front of the end stop (6) in the retraction direction of the dosing plunger (4).

12. Dosing system according to one of the preceding claims, **characterized in that** the end stop (6) is fashioned separate from the dosing sleeve (3) and is secured preferably by form fitting, preferably by a locking connection, to the dosing sleeve (3).

13. Dosing system according to one of the preceding claims, **characterized in that** the end stop (6) is arranged adjacent to a second end (20) of the dosing sleeve (3), which is opposite to the at least one communication opening (7).

14. Dosing system according to one of the preceding claims, **characterized in that** the dosing plunger (4) is axially movable relative to the retraction limiting element (5) when the retraction limiting element (5) is in the outer position.

15. Dosing system according to one of the preceding claims, **characterized by** a retaining mechanism operating between the dosing plunger (4) and the retraction limiting element (5) which limits the outward radial displacement of the retraction limiting element (5).

## Revendications

1. Système de dosage pour le réglage d'un volume de dosage d'un milieu coulant, comprenant :
a) un axe longitudinal médian (1),
b) un manchon de dosage (3) pourvu d'au moins un orifice de communication (7) pour la communication avec l'environnement, et
c) un corps de piston de dosage (4), qui est guidé de manière à pouvoir coulisser entre une position tirée vers le haut et une position avancée dans le manchon de dosage (3) en direction de l'axe longitudinal médian (1) pour la réception et la distribution du milieu par l'intermédiaire dudit au moins un orifice de communication (7),
**caractérisé par**
d) un élément de limitation de traction vers le haut (5) destiné à limiter une course maximale de traction vers le haut du corps de piston de dosage (4) en direction de l'axe longitudinal médian (1),
i) dans lequel l'élément de limitation de traction vers le haut (5) peut être bloqué axialement sur le corps de piston de dosage (4) dans au moins une position,
ii) dans lequel l'élément de limitation de traction vers le haut (5) peut être déplacé vis-à-vis du corps de piston de dosage (4) de manière radiale par rapport à l'axe longitudinal médian (1) entre une position radialement intérieure, bloquée axialement sur le corps de piston de dosage (4) et une position radialement extérieure, et
iii) dans lequel dans la position radialement extérieure de l'élément de limitation de traction vers le haut (5), le corps de piston de dosage (4) peut être déplacé par rapport au manchon de dosage (3) et à l'élément de limitation de traction vers le haut (5) en direction de l'axe longitudinal médian (1), et
iv) dans lequel l'élément de limitation de traction vers le haut (5), dans la position intérieure, est agencé entièrement à l'intérieur du manchon de dosage (3), et
e) une butée (6) agencée sur le manchon de dosage (3) et destinée à interagir avec l'élément de limitation de traction vers le haut (5) dans la position tirée vers le haut du corps de piston de dosage (4).

2. Système de dosage selon la revendication 1, **caractérisé en ce qu'**une étendue maximale de l'élément de limitation de traction vers le haut (5) perpendiculairement à l'axe longitudinal médian (1) est inférieure à un diamètre intérieur DI du manchon de dosage (3).

3. Système de dosage selon l'une quelconque des revendications précédentes, **caractérisé en ce que** l'élément de limitation de traction vers le haut (5), dans la position intérieure, est bloqué axialement localement sur le corps de piston de dosage (4) par liaison par coopération de formes, de préférence au moyen d'une liaison d'encliquetage.

4. Système de dosage selon la revendication 3, **caractérisé en ce que** plusieurs premiers moyens de coopération de forme (29) sont prévus sur le corps de piston de dosage (4) et sont espacés les uns des autres dans la direction de l'axe longitudinal médian (1), dans lequel au moins un deuxième élément de coopération de formes (34) destiné à interagir par coopération de formes avec au moins un des premiers moyens de coopération de forme (29) est prévu sur l'élément de limitation de traction vers le haut (5).

5. Système de dosage selon l'une quelconque des revendications précédentes, **caractérisé en ce que** l'élément de limitation de traction vers le haut (5), dans la position intérieure, est bloqué localement sur le corps de piston de dosage (4) de manière non détachable.

6. Système de dosage selon l'une quelconque des revendications précédentes, **caractérisé en ce que** l'élément de limitation de traction vers le haut (5), lorsqu'il se trouve dans la position extérieure, lors du coulissement axial du corps de piston de dosage (4) pour émettre une information par rapport à au moins une position, interagit avec le corps de piston de dosage (4) pour bloquer axialement l'élément de limitation de traction vers le haut (5) sur le corps de piston de dosage (4).

7. Système de dosage selon l'une quelconque des revendications précédentes, **caractérisé en ce qu'**une fenêtre d'actionnement (47) pour l'élément de limitation de traction vers le haut (5) est prévue latéralement dans le manchon de dosage (3).

8. Système de dosage selon la revendication 7, **caractérisé en ce que** l'élément de limitation de traction vers le haut (5), dans la position extérieure, traverse la fenêtre d'actionnement (47) à la façon d'un verrou et se termine de préférence à l'extérieur sensiblement à fleur avec le manchon de dosage (3).

9. Système de dosage selon la revendication 7 ou 8, **caractérisé en ce que** l'élément de limitation de traction vers le haut (5) présente en direction de l'axe longitudinal médian (1) une étendue qui correspond sensiblement à l'étendue de la fenêtre d'actionnement (47) en direction de l'axe longitudinal médian (1).

10. Système de dosage selon l'une quelconque des revendications 7 à 9, **caractérisé en ce que** l'élément de limitation de traction vers le haut (5) présente perpendiculairement à l'axe longitudinal médian (1) une largeur qui correspond sensiblement à la largeur de la fenêtre d'actionnement (47) perpendiculairement à l'axe longitudinal médian (1).

11. Système de dosage selon l'une quelconque des revendications 7 à 10, **caractérisé en ce que** la fenêtre d'actionnement (47) est agencée au voisinage de la butée (6), de préférence dans la direction de traction vers le haut du corps de piston de dosage (4) juste devant la butée (6).

12. Système de dosage selon l'une quelconque des revendications précédentes, **caractérisé en ce que** la butée (6) est formée séparément du manchon de dosage (3) et est fixée au manchon de dosage (3) de préférence par coopération de formes, plus préférentiellement par liaison par encliquetage.

13. Système de dosage selon l'une quelconque des revendications précédentes, **caractérisé en ce que** la butée (6) est agencée au voisinage d'une deuxième extrémité (20) du manchon de dosage (3), qui se situe à l'opposé dudit au moins un orifice de communication (7).

14. Système de dosage selon l'une quelconque des revendications précédentes, **caractérisé en ce que** le corps de piston de dosage (4) peut être déplacé axialement par rapport à l'élément de limitation de traction vers le haut (5) lorsque l'élément de limitation de traction vers le haut (5) se trouve dans la position extérieure.

15. Système de dosage selon l'une quelconque des revendications précédentes, **caractérisé par** un dispositif de retenue qui agit entre le corps de piston de dosage (4) et l'élément de limitation de traction vers le haut (5) et qui limite le déplacement radial de l'élément de limitation de traction vers le haut (5) vers l'extérieur.
